(19) Europäisches Patentamt
European Patent Office
Office européen des brevets

(11) Publication number: 0 002 341 B1

(12) **EUROPEAN PATENT SPECIFICATION**

(45) Date of publication of patent specification: 20.01.82

(21) Application number: 78300671.1

(22) Date of filing: 27.11.78

(51) Int. Cl.$^3$: C 07 C 65/10, C 07 C 101/50, C 07 C 149/40, C 07 C 163/00, C 07 D 239/48, A 61 K 31/60

(54) **Complexes of bivalent copper, methods of preparation thereof and compositions containing said complexes.**

(30) Priority: 28.11.77 AU 2584/77
16.08.78 AU 5533/78

(43) Date of publication of application: 13.06.79 Bulletin 79/12

(45) Publication of the grant of the European patent: 20.01.82 Bulletin 82/3

(84) Designated Contracting States: BE CH DE FR GB LU NL SE

(56) References cited:

US - A - 3 884 949

Chemical Abstracts vol. 69, no. 25, 1968, Columbus, Ohio, USA
J. KRATSMAR SMOGRAVIC et al.: "Copper (II) salicylate and o-cresotate complexes with ethanol"
page 10 573, abstract no. 113 013z.

Chemical Abstracts vol. 73, no. 22, 1970, Columbus, Ohio, USA
O. HULKOVA et al.: "Binding of binuclear copper (II)-salicylate and copper (II)-2,3-cresotate complexes"
page 461, abstract no. 115 882p.

(73) Proprietor: Boettcher, Barry
12 Mahogany Drive
New Lambton New South Wales, 2305 (AU)

(73) Proprietor: Walker, William Raymond
University of Newcastle
New South Wales, 2308 (AU)

(73) Proprietor: Whitehouse, Michael Wellesley
Department of Experimental Pathology Australian National University
Canberra A.C.T., 2600 (AU)

(72) Inventor: Boettcher, Barry
12 Mahogany Drive
New Lambton New South Wales, 2305 (AU)
Inventor: Walker, William Raymond
University of Newcastle
New South Wales, 2308 (AU)
Inventor: Whitehouse, Michael Wellesley
Department of Experimental Pathology Australian National University
Canberra A.C.T., 2600 (AU)

(74) Representative: Burnside, Michael et al,
c/o Michael Burnside & Partners 2 Serjeants' Inn
Fleet Street
London EC4Y 1HL (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 002 341 B1

(56) References cited:

Chemical Abstracts vol. 84, no. 7, 1976, Columbus, Ohio, USA
J. R. J. SORENSON "Copper chelates as possible active forms of the antiarthritic agents" pages 14 to 15, abstract no. 38 591r.

Chemical Abstracts vol. 84, no. 23, 1976, Columbus, Ohio, USA
J. R. J. SORENSON "The anti-inflammatory activity of some copper chelates" page 31, abstract no. 159 683f.

Chemical Abstracts vol. 85, no. 17, 1976, Columbus, Ohio, USA
J. R. J. SORENSON "Some copper coordination compounds and their antiinflammatory and antiulcer activities" page 40, abstract no. 116 777t.

# Complexes of bivalent copper, methods of preparation thereof and compositions containing said complexes.

This invention relates to novel anti-inflammatory copper complexes and to anti-inflammatory compositions and processes utilizing such complexes.

Many studies of the role of copper, and especially complexes of copper, in the treatment of inflammatory diseases have been reported in recent years. Certain recent studies describe the use of parenterally administered copper complexes, and particularly copper (II) salicylate or anthranilate, in the treatment of rheumatic disease (Sorenson and Hangarter, Inflammation *2*, 217—238 (1977)) and (Sorenson, Inflammation *1*, 317—331 (1976)).

The prior art describes a wide variety of copper-salicylate complexes prepared by the reaction of inorganic copper (II) salts with salicylic acid in aqueous solution. The specific complex formed in aqueous solution has been found to depend on the pH of the aqueous solution and both neutral complexes such as the pale blue crystalline bis (salicylato) copper (II) tetrahydrate

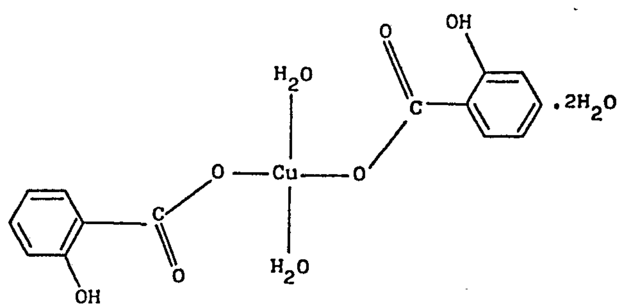

and anionic complexes such as the olive green sodium salicylatocuprate (II)

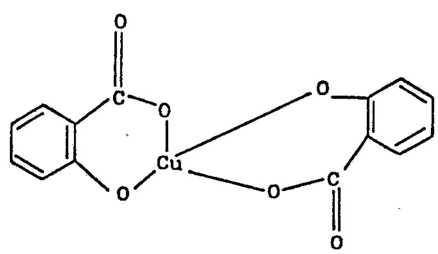

are known in the art.

It is an object of this invention to provide novel neutral copper complexes suitable for the treatment of inflammatory diseases of animals prepared by the reaction of copper compounds and substituted benzoic acids in non-aqueous solvents.

Accordingly in one embodiment the invention provides neutral copper (II) complexes of the formula $Cu[C_6H_4(X)COO]_2$ ROH wherein ROH represents an alkanol, preferably a $C_1$ to $C_6$ aliphatic alkanol, and wherein X can be located in any position and is SH, SeH, or $NHR^1$ where $R^1$ is

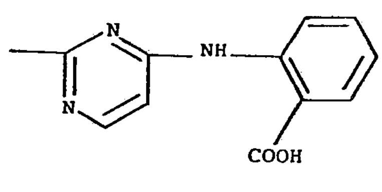

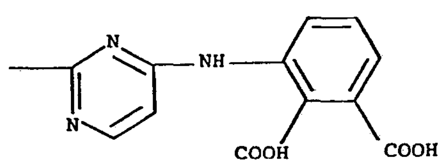

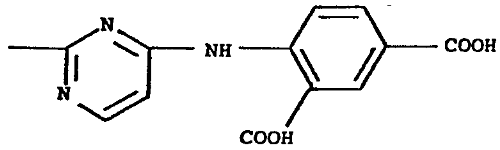

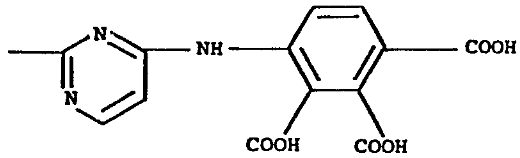

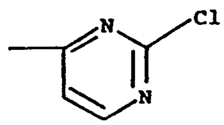

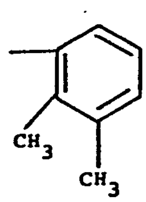

Although in no way wishing to be bound by theory, as a result of physico-chemical studies the complexes of the invention are believed to have the following dimeric structure:

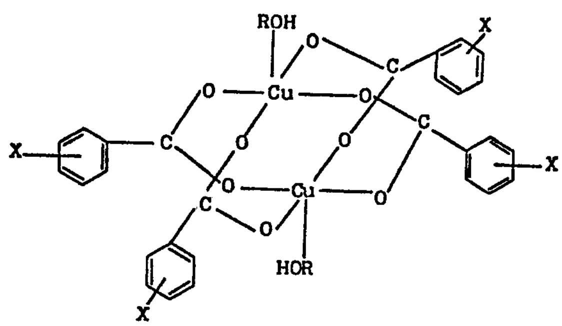

The preferred compound according to the invention is the copper salicylate complex which is believed to have the formula:

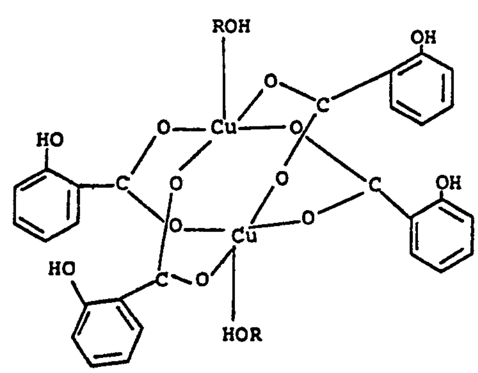

(II)

The compounds of the invention may be prepared by reacting inorganic copper (II) compounds, for example copper salts or cupric hydroxide, with the appropriate substituted benzoic acid in the presence of an alkanol. For example, reaction of cupric hydroxide with salicylic acid in anhydrous

ethanol gives the complex of formula $Cu[C_6H_4(OH)COO]_2$. $C_2H_5OH$, hereinafter referred to by the formula Cu[H Sal]$_2$. $C_2H_5$ OH, which forms deep green crystals when crystallised from anhydrous ethanol containing excess (approx. 5M) salicylic acid.

The compounds of the invention have been found to be particularly useful in the treatment of inflammatory diseases in animals.

The compounds of the invention have proved particularly effective in alleviating the symptoms of inflammatory diseases such as rheumatic disease, arthritis and rheumatoid arthritis when applied topically to the animal.

Preferably the compounds of the invention are applied in the form of a composition comprising a compound of the invention in admixture with a pharmaceutically acceptable carrier. Therefore, in yet a further aspect the invention provides pharmaceutical anti-inflammatory compositions comprising a compound of the invention as hereinbefore defined and a pharmaceutically acceptable non-aqueous carrier therefor.

The compositions are preferably suitable for topical application to animals to be treated and therefore may be in the form of a gel, an ointment, a paste, a cream or a lotion. Compositions comprising a compound of the invention in solution are preferred as they are more efficient in perfusing the skin.

The compounds of the invention have limited stability in the presence of water and therefore the compositions comprise a non-aqueous carrier. More preferred compositions comprise a compound of the invention dissolved in a non-aqueous lipophilic carrier. Suitable carriers include monohydric, dihydric and trihydric alkanols such as, for example: short chain ($C_1$ to $C_{10}$) and long chain $C_{12}$ to $C_{20}$ alcohols including methanol, ethanol, propanol, butanol and cetyl alcohol; dihydric alcohols such as diethylene glycol; and polyhydric alcohols such as glycerol.

Even more preferred compositions which have been shown to have an indefinite shelf life, comprise a compound of the invention in solution in a liquid carrier comprising an alkanol, glycerol and salicylic acid.

The amount of the compound of the invention employed in the compositions will depend to a large extent on the inflammatory condition being treated. However, as a general rule the compositions may comprise from 0.1 to 15% w/v of the copper compounds of the invention and preferably from 1 to 7% w/v.

As previously indicated the compounds of the invention have proved particularly useful in the alleviation of the symptoms of inflammatory disease when applied topically to the animal in the form of a pharmaceutical composition as hereinbefore defined. The compounds are believed to be efficacious in such treatments because of their ready penetration of the skin. Furthermore, the compounds are believed to offer particular advantages in the treatment of inflammatory diseases of humans because of the non-toxic nature of the compounds and evidence that the compounds are readily cleared from the treated animal as indicated by the limited duration of the anti-inflammatory effect of the compounds.

The compositions may comprise, in addition to one or more compounds of the invention, other pharmaceutically active ingredients including other anti-inflammatory agents and conventional pharmaceutical excipients known in the art.

Example 1

Preparation of Cu[H Sal]$_2$. $C_2H_5OH$.

Freshly prepared $Cu(OH)_2$ (0.97 g) was added to a solution of salicylic acid (6.9 g) in anhydrous ethanol (50 ml). The suspension was then refluxed on a water bath until all the copper hydroxide had reacted. The resultant deep-green-coloured solution was then filtered and green crystals were deposited on cooling. These were filtered off, washed with cold alcohol and air dried. Yield 3.2 g (Found: Cu, 16.6; C, 50.2; H, 4.2; $CuC_{16}H_{16}O_7$ requires Cu, 16.6; C, 50.1; H, 4.2%.)

Example 2

Preparation of Cu[H Sal]$_2$. $CH_3OH$ and Cu[H Sal]$_2$. $C_3H_7OH$.

Cu [H Sal]$_2$. $CH_3OH$, analysed as Cu $C_{15}H_{14}O_7$, and Cu [H Sal]$_2$. $C_3H_7OH$, analysed as Cu $C_{17}H_{18}O_7$, were prepared following the procedure described in Example 1 and substituting for anhydrous ethanol, anhydrous methanol and anhydrous *n*-propanol respectively.

Example 3

This example demonstrates the preparation of a composition comprising Cu[H Sal]$_2$. $C_2H_5OH$.

Freshly prepared cupric hydroxide (1.0 g) was added to a solution of salicylic acid (7 g) in anhydrous ethanol (80 ml). The suspension was heated under reflux until all the cupric hydroxide had reacted. The resultant deep-green-coloured solution was filtered to remove any insoluble polymeric copper salicylate and glycerol (20 ml) was added to the filtrate.

The deep-green *c.* 0.1 M Cu [H Sal]$_2$. $C_2H_5OH$ solution obtained by this procedure was found to be stable for an indefinite period and was used in the tests described in Examples 4 and 5.

Example 4

This example demonstrates the percutaneous absorption of the compound Cu[H Sal]$_2$. $C_2H_5OH$.

Male Wistar rats (average weight 250 g) were anaesthetised with diethyl ether and an area of *c* 20 sq. cm. was shaven high on their backs. The Cu[H Sal]$_2$. $C_2H_5OH$ composition prepared according to example 3 was then applied to each rat (*c*. 20 ml per 3 rats). The rats were then returned to their cages and after two days their urine was collected and compared with the urine of control, untreated, rats following the procedure below.

Urine (5 ml) was acidified with 2M $H_2SO_4$ and shaken with ethyl acetate (2 ml). After centrifuging the ethyl acetate layer was analysed by thin layer chromatography on silica gel (solvent: benzene, diethyl ether; glacial acetic acid; methanol; 120:60:18:1 by volume) along with samples of salicylic acid, salicyluric acid, gentisic acid and ethyl salicylate.

The results are shown in Figure 1 which is a diagrammatic representation of a chromatogram run under the conditions described above with solvent flow from origin line, represented by the points of application of the samples 1 to 9, in the direction of the arrow shown and wherein:

1 and 9 represent the points of application of a sample of a mixture of salicyluric acid (a) and salicyclic acid (b) chromatographed for comparative purposes;

2 and 8 represent the points of application of a sample of the urine extract of rats treated with the Cu H[Sal]$_2$. $C_2H_5OH$ composition;

3 and 7 represent the points of application of a sample of the urine extract of control, untreated, rats;

4 and 6 represent the points of application of a sample of a mixture of gentisic acid (c) and ethyl salicylate (b) chromatographed for comparative purposes; and

5 represents the point of application of a sample of the Cu[H Sal]$_2$. $C_2H_5OH$ composition used to treat the rats.

The chromatogram clearly shows the presence of salicylic acid and gentisic acid in the urine of the Cu[H Sal]$_2$. $C_2H_5OH$ treated rats indicating that the composition has perfused the skin of the rats.

Example 5

This example demonstrates the effectiveness of topical application of the compound Cu[H Sal]$_2$. $C_2H_5OH$ in alleviating the symptoms of artificially induced inflammation in rats.

Male Wistar rats (average weight 250 g) were anaesthetised with diethyl ether and an area of *c*. 20 sq. cm was shaven high on their backs. The rats were then separated into 7 groups. Group 1, the control rats were not treated. Group 2 rats were treated by the application of a mixture of alcohol and glycerol (4:1) the carrier used for the compound Cu[H Sal]$_2$. $C_2H_5OH$. Group 3 rats were treated with a 7% w/v solution of salicylic acid in a mixture of alcohol and glycerol (4:1). Group 4, 5, 6 and 7 rats were treated with amounts of Cu[H Sal]$_2$. $C_2H_5OH$ composition prepared according to example 3 containing 154 mg, 231 mg, 462 mg and 770 mg respectively of Cu[H Sal]$_2$. $C_2H_5OH$.

About 6 hours after the topical treatment of the rats inflammation was induced in a foot of each rat in each of the groups by the injection of 0.1 ml of 1% saline solution of sodium carrageenan into a foot pad of each rat. The inflammation of the feet of the rats in each group was then monitored by the swelling of the feet with a micrometer screw-gauge.

The results are shown in Figure 2 wherein the curves represent the increase in paw thickness of the rats with time after the injection of the inflammatory agent. The curves 1 to 7 represent the swelling of the paws of the rats of groups 1 to 7 respectively.

Figure 2 clearly shows the anti-inflammatory effect of the topically applied compound Cu[H Sal]$_2$. $C_2H_5OH$.

Example 6

Preparation of $Cu[C_6H_4(SH)COO]_2$. $C_2H_5OH$.

This compound was prepared as described in Example 1, but substituting 2-thiobenzoic acid (7.7 g) for the salicylic acid. Yield 2.9 g. (Found: Cu 15.6; C 45.9; H 3.6 $CuC_{16}H_{16}O_5S_2$ requires Cu 15.4; C 46.2; H 3.9%).

Example 7

Preparation of $Cu[C_6H_4(SeH)COO]_2$. $C_2H_5OH$.

This compound was prepared as described in Example 1, but substituting 2-selenibenzoic acid (10.0 g) for the salicylic acid. Yield 8.1 g. (Found: Cu 12.3; C 38.1; H 3.5. $CuC_{16}H_{16}O_5Se_2$ requires Cu 12.6; C 37.7; H 3.1%).

Example 8

Preparation of $Cu[C_6H_4(NH_2)COO]_2.C_2H_5OH$.

This compound was prepared as described in Example 1, but substituting 2-aminobenzoic acid (6.9 g) for the salicylic acid. Yield 5.6 g. (Found: Cu 16.4; C 50.6; H 5.0. $CuC_{16}H_{18}O_5N_2$ requires Cu 16.8; C 50.3; H 4.7%).

Example 9

Preparation of $Cu_2[C_6H_4.CO_2.NH.C_4N_2H_2.NH.C_7H_4O_2]$. $C_2H_5OH$.

This compound was prepared as described in Example 1, but substituting 2-[2-carboxyphenyl]amino-4-[2-carboxyphenyl] amino-pyrimidine (16.8 g) for salicylic acid. Yield 13.3 g. (Found: Cu 24.5; C 46.2; H 3.2. $Cu_2C_{20}H_{18}O_5N_4$ requires Cu 24.5; C 46.0; H 3.5%).

Example 10

Preparation of $Cu_2[C_6H_4.CO_2.NH.C_4N_2H_2.NH.C_8H_4O_4]$. $C_2H_5OH$.

This compound was prepared as described in Example 1, but substituting 2-[2-carboxyphenyl]amino-4-[2,3-dicarboxyphenyl]amino-pyrimidine (19.0 g) for the salicylic acid. Yield 14.0 g. (Found: Cu 22.4; C 44.7; H 3.3. $Cu_2C_{21}H_{18}O_7N_4$ requires Cu 22.6; C 44.5; H 3.2%).

Example 11

Preparation of $Cu_2[C_6H_4.CO_2.NH.C_4N_2H_2.NH.C_8H_4O_4]$. $C_2H_5OH$.

This compound was prepared as described in Example 1, but substituting 2-[2-carboxyphenyl]amino-4-[2,4-dicarboxyphenyl]amino-pyrimidine (19.0 g) for the salicylic acid. Yield 15.3 g. (Found: Cu 24.7; C 45.8; H 3.7. $Cu_2C_{21}H_{18}O_7N_4$ requires Cu 24.5; C 46.0; H 3.5%).

Example 12

Preparation of $Cu[C_6H_4.CO_2.C_4N_2H_2Cl]_2.C_2H_5OH$.

This compound was prepared as described in Example 1, but substituting 4-(2-carboxyphenyl)amino-2-chloro-pyrimidine (11.7 g) for the salicylic acid. Yield 9.5 g. (Found: Cu 12.5; C 56.5; H 3.9. $CuC_{24}H_{18}O_5N_4$ requires Cu 12.7; C 56.9; H 3.6%).

Example 13

Preparation of $Cu[C_6H_4.CO_2.NH.C_8H_9]_2.C_2H_5OH$.

This compound was prepared as described in Example 1, but substituting 2-[2,3-dimethylphenyl]aminobenzoic acid (6.8 g) for the salicylic acid. Yield 5.5 g. (Found: Cu 11.2; C 64.9; H 5.5. $CuC_{32}H_{34}O_5N_2$ requires Cu 10.9; C 65.1; H 5.8%).

## Claims

1. Neutral copper (II) complexes characterized in that they have the formula $Cu[C_6H_4(X)COO]_2$ ROH wherein ROH represents an alkanol, and wherein X can be located in any position and is SH, SeH and $NHR^1$ where $R^1$ is

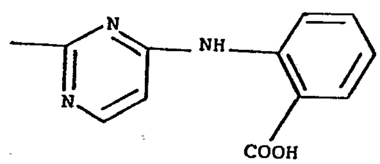

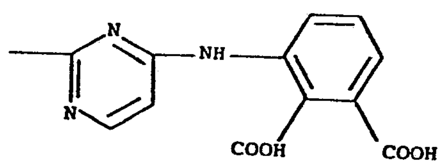

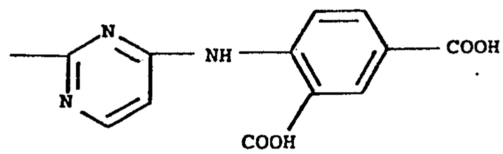

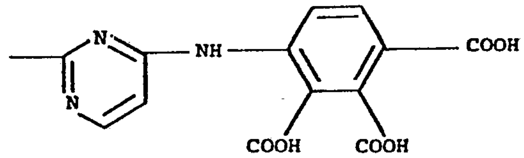

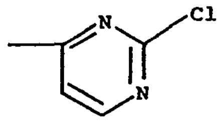

or

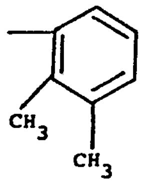

2. Complexes according to Claim 1 characterized in that the alkanol is a $C_1$ to $C_6$ aliphatic alkanol.

3. Complexes according to Claim 1 or Claim 2 characterized in that they have the formula

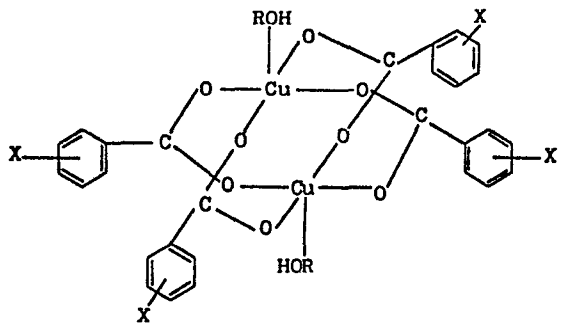

4. A method of preparing complexes as claimed in any of Claims 1 to 3 characterized in that it comprises reacting an inorganic copper (II) compound with a substituted benzoic acid having the formula

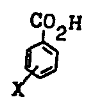

wherein X has the meaning given in Claim 1, in the presence of an alkanol.

5. A method according to Claim 4 wherein the reaction is carried out under anhydrous conditions.

6. A method according to Claim 4 or Claim 5, characterized in that the copper (II) compound is a salt or cupric hydroxide.

7. A method according to any one of Claims 4 to 6, characterized in that the alkanol is a $C_1$ to $C_6$ aliphatic alkanol.

8. A pharmaceutical composition characterized in that it comprises a neutral copper (II) complex of formula $Cu[C_6H_4(X)COO]_2$ ROH wherein ROH represents an alkanol, and wherein X can be located in any position and is OH, SH, SeH, $NH_2$ or $NHR^1$ where $R^1$ is

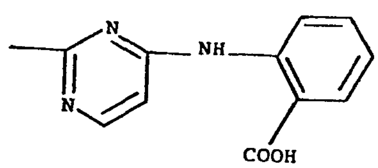

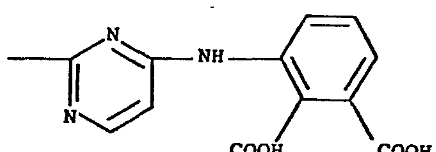

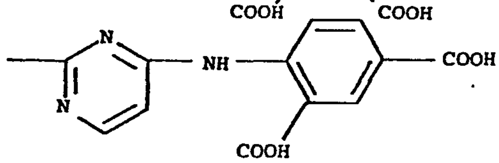

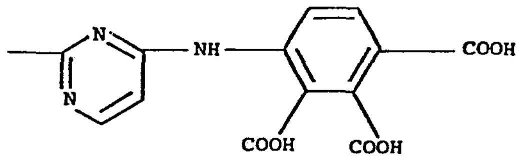

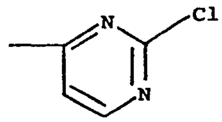

or

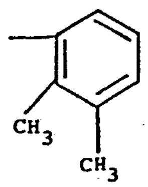

and a pharmaceutically acceptable non-aqueous, carrier.

9. A composition according to Claim 8, in the form of an anhydrous gel, ointment, paste, cream or lotion for topical application.

10. A composition according to Claim 8 in the form of a solution comprising the copper complex, a molar excess of salicylic acid and ethanol.

11. A composition according to Claim 8, characterized in that the carrier comprises an alkanol, glycerol and salicylic acid.

12. A composition according to any one of Claims 8 to 11 characterized in that it contains 0.1 to 15% w/v of active ingredient.

**Patentansprüche**

1. Neutrale Kupfer-(II)-Komplexe, dadurch gekennzeichnet, daß sie die Formel $Cu[C_6H_4(X)COO]_2ROH$ aufweisen, in der ROH ein Alkanol bedeutet und X in jeder beliebigen Position angeordnet sein kann, wobei X SH, SeH oder $NHR^1$ bedeutet und $R^1$

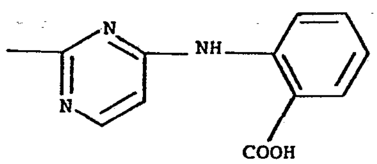

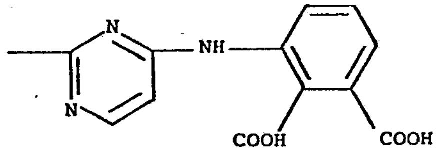

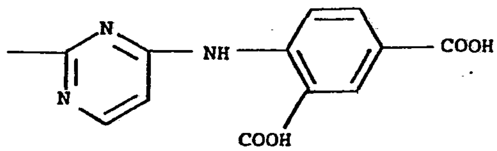

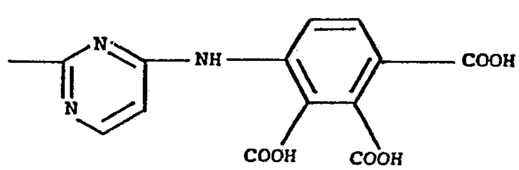

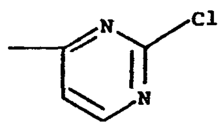

oder

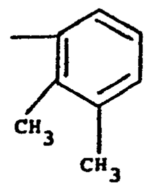

sein kann.

2. Komplexe nach Anspruch 1, dadurch gekennzeichnet, daß das Alkanol ein aliphatischer $C_1$ bis $C_6$-Alkohol sein kann.

3. Komplexe nach Anspruch 1 oder 2, dadurch gekennzeichnet, daß sie die folgende allgemeine Formel aufweisen:

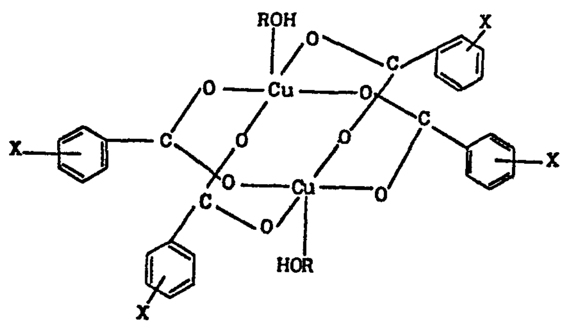

4. Verfahren zur Herstellung der Komplexe nach einem der Ansprüche 1 bis 3, gekennzeichnet durch Umsetzung einer anorganischen Kupfer-(II)-Verbindung mit einer substituierten Benzoiesäure der Formel

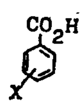

wobei X wie in Anspruch 1 definiert ist, in Gegenwart eines Alkanols.

5. Verfahren nach Anspruch 4, dadurch gekennzeichnet, daß man die Reaktion unter wasserfreien Bedingungen durchführt.

6. Verfahren nach Anspruch 4 oder 5, dadurch gekennzeichnet, daß die Kupfer-(II)-Verbindung ein Salz oder Kupferhydroxid ist.

7. Verfahren nach einem der Ansprüche 4 bis 6, dadurch gekennzeichnet, daß das Alkanol ein aliphatisches $C_1$ bis $C_6$-Alkanol ist.

8. Pharmazeutische Zusammensetzung, dadurch gekennzeichnet, daß sie einen neutralen Kupfer-(II)-Komplex der Formel $Cu[C_6H_4(X)COO]_2$ ROH, in der ROH ein Alkanol bedeutet und X in jeder beliebigen Position angeordnet sein kann, wobei X SH, SeH oder $NHR^1$ bedeutet und $R^1$

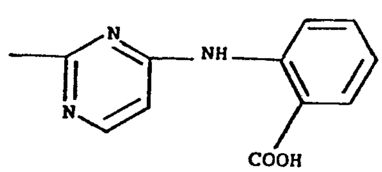

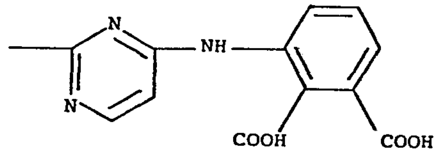

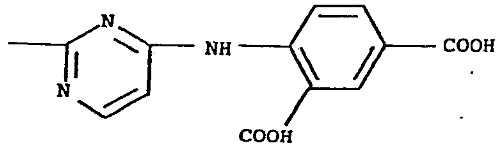

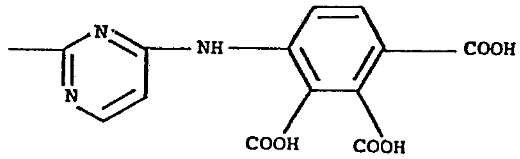

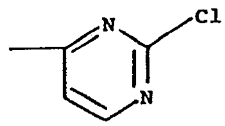

oder

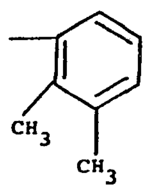

sein kann, und einen pharmazeutisch verträglichen nicht-wässrigen Träger umfasst.

9. Zusammensetzung nach Anspruch 8, dadurch gekennzeichnet, daß sie in Form eines wasserfreien Gels, einer Tinktur, einer Paste, einer Creme oder einer Lotion für die topische Anwendung vorliegt.

10. Zusammensetzung nach Anspruch 8, dadurch gekennzeichnet, daß sie in Form einer Lösung vorliegt, welche den Kupferkomplex, einen molaren Überschuß an Salicylsäure und Ethanol umfasst.

11. Zusammensetzung nach Anspruch 8, dadurch gekennzeichnet, daß der Träger einen Alkohol, Glycerin und Salicylsäure umfasst.

12. Zusammensetzung nach einem der Ansprüche 8 bis 11, dadurch gekennzeichnet, daß sie 0,1 bis 15 Gew% des Wirkstoffs pro Volumen enthält.

## Revendications

1. Complexes neutres de cuivre divalent, caractérisé en ce qu'ils présentent la formule $Cu[C_6H_4(X)COO]_2ROH$, dans laquelle
ROH représente un alcanol et
X peut être placé en toute position et est SH, SeH ou $NHR^1$, ou $R^1$ est

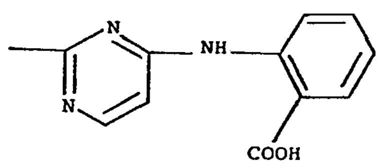

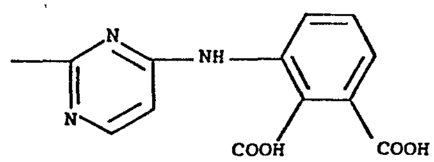

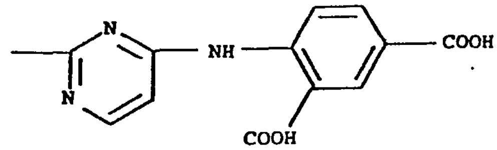

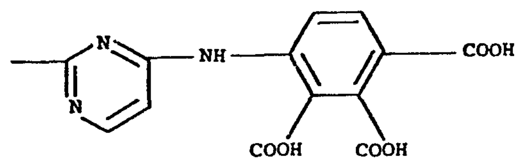

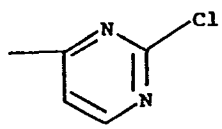

ou

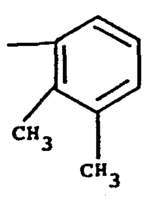

2. Complexes selon la revendication 1, caractérisé en ce que l'alcanol est un alcanol aliphatique $C_1$ à $C_6$.

3. Complexes selon la revendication 1 ou la revendication 2, caractérisé en ce qu'ils présentent la formule

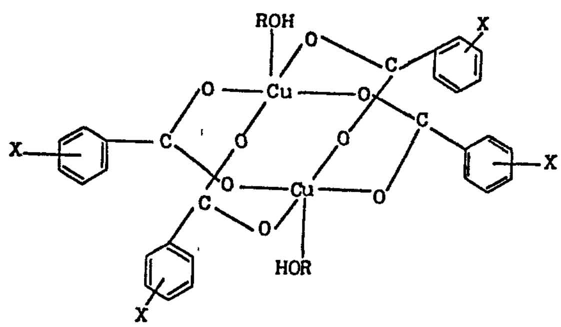

4. Un procédé pour préparer des complexes tels que spécifiés sous l'une quelconque des revendications 1 à 3, caractérisé en ce qu'il comporte l'étape de faire réagir un composé non organique de cuivre divalent avec un acide benzoïque substitué ayant la formule:

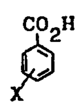

dans laquelle X a la même signification que dans la revendication 1, en présence d'un alcanol.

5. Un procédé selon la revendication 4, caractérisé en ce que la réaction est effectuée dans des conditions anhydres.

6. Un procédé selon la revendication 4 ou la revendication 5, caractérisé en ce que le composé de cuivre divalent est un sel ou un hydroxyde cuivrique.

7. Un procédé selon l'une quelconque des revendications 4 à 6, caractérisé en ce que l'alcanol est un alcanol aliphatique $C_1$ à $C_6$.

8. Une composition pharmaceutique, caractérisée en ce qu'elle comporte un complexe neutre de cuivre divalent de formule $Cu[C_6H_4(X)COO]_2$ ROH, dans laquelle
ROH représente un alcanol et
X peut être placé en toute position et est SH, SeH, $NH_2$ ou $NHR^1$, où $R^1$ est

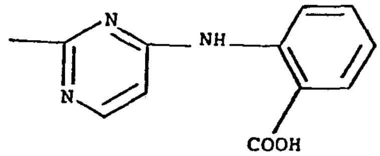

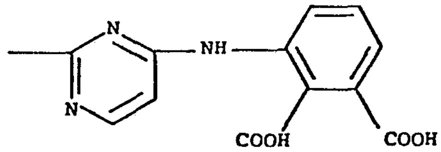

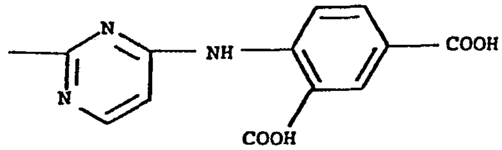

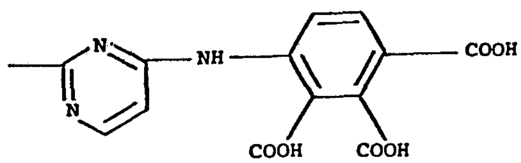

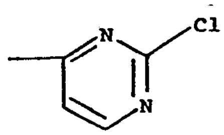

ou

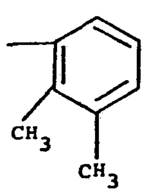

et un support non aqueux acceptable pharmaceutiquement.

9. Une composition selon la revendication 8, sous la forme d'un gel anhydre, onguent, pâte, crème ou lotion pour application locale.

10. Une composition selon la revendication 8, sous la forme d'une solution comprenant le complexe cuivrique, un excès molaire d'acide salicylique et de l'éthanol.

11. Une composition selon la revendication 8, caractérisée en ce que le support comporte un alcanol, du glycérol et de l'acide salicylique.

12. Une composition selon l'une quelconque des revendications 8 à 11, caractérisée en ce qu'elle comporte de 0,1 à 15% en poids/volume de produit actif.

Fig.1

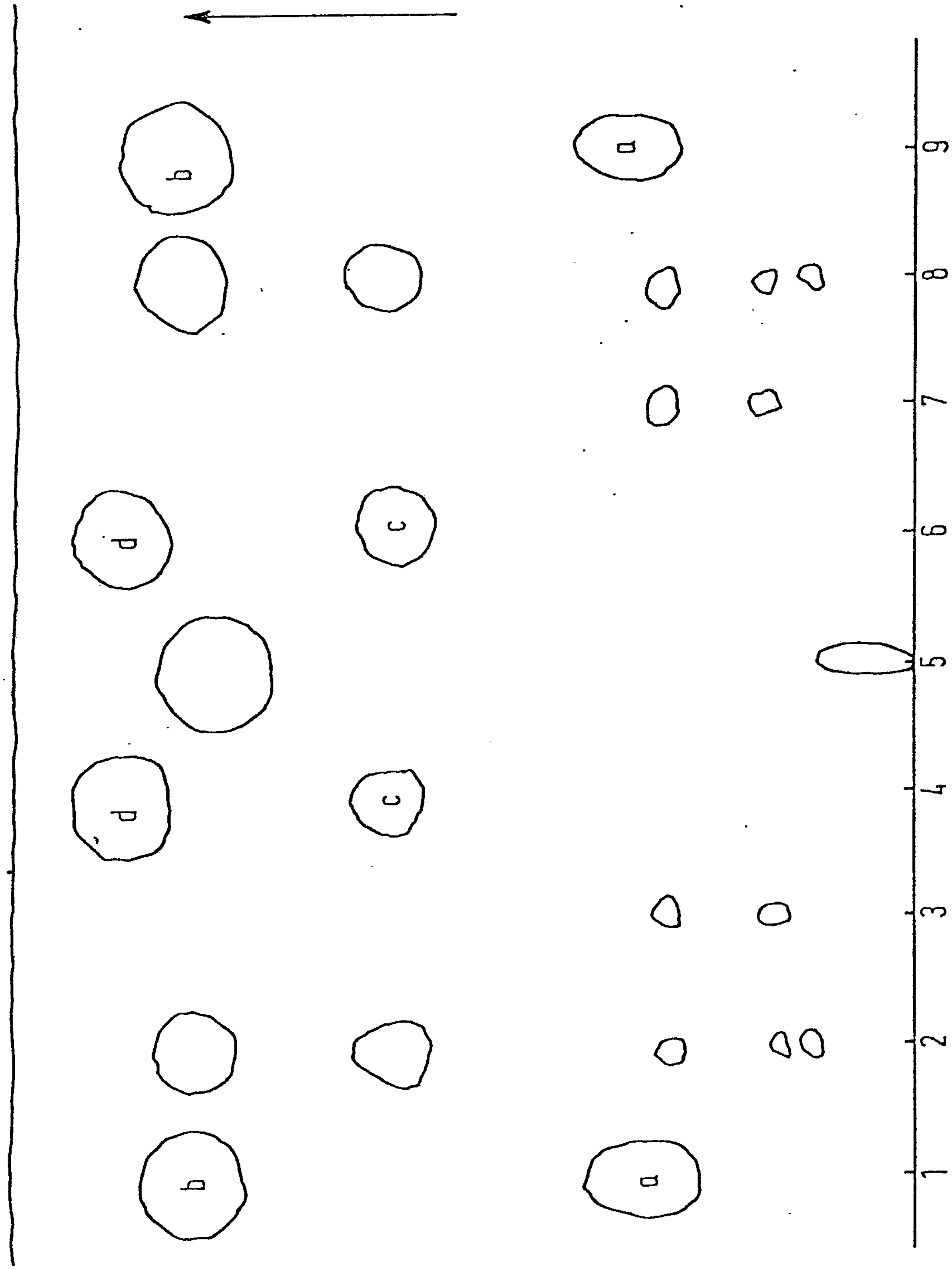

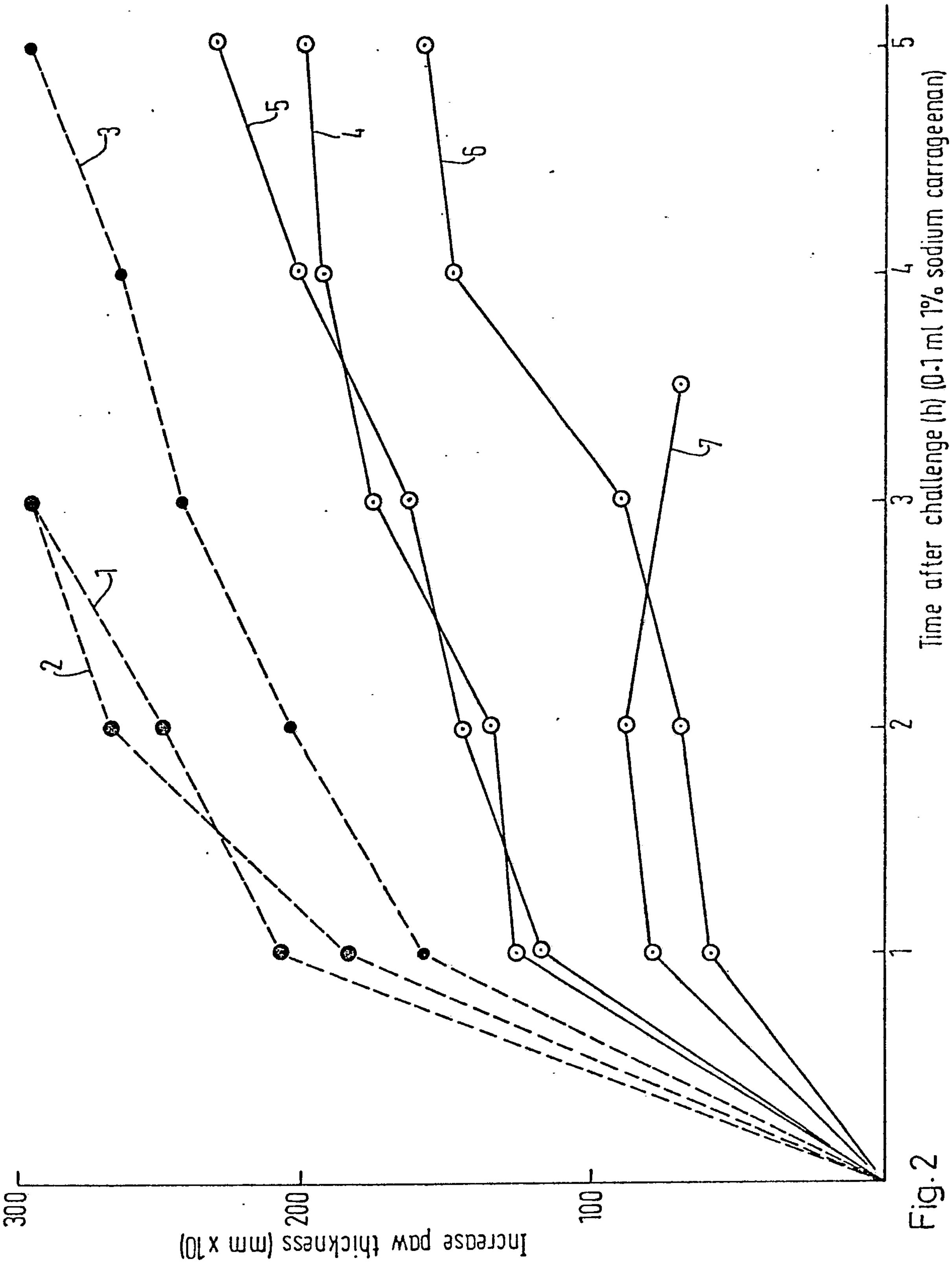

300
200
100
Increase paw thickness (mm x10)
1
2
3
4
5
Time after challenge (h) (0·1 ml 1% sodium carrageenan)
1
2
3
4
5
6
7


Fig. 2